# EUROPEAN PATENT SPECIFICATION

(11) **EP 0 858 993 B1**
(45) Date of publication and mention of the grant of the patent: **25.06.2003**
(21) Application number: 98106734.1
(22) Date of filing: 24.04.1992
(51) Int. Cl.: C07C 227/02, C07C 227/16, C07C 229/52

(54) **Method of producing keto acids**
Verfahren zur Herstellung von Ketosäuren
Procédé pour la fabrication d'acides cétoniques

(30) Priority: 25.04.1991 JP 9590191; 11.03.1992 JP 5288992
(43) Date of publication of application: 19.08.1998
(62) Divisional of application: 92303748.5
(73) Proprietor: Mitsui Chemicals, Inc., Tokyo 100 (JP)
(72) Inventor: Kondo, Masahiro, Kuga-gun, Yamaguchi (JP); Tanaka, Michio, Kuga-gun, Yamaguchi (JP); Sakamoto, Naoya, Kuga-gun, Yamaguchi (JP); Ooyoshi, Hajime, Kuga-gun, Yamaguchi (JP)
(74) Representative: Cresswell, Thomas Anthony

(56) References cited:
- DE-A- 3 407 369
- DE-A- 3 415 331
- DE-C- 85 931
- GB-A- 1 182 743
- GB-A- 1 306 263
- CHEMICAL ABSTRACTS, vol. 108, no. 4, 25 January 1988 Columbus, Ohio, US; abstract no. 23350b, XP002065439 & JP 62 070 350 A (KANZAKI PAPER MFG CO LTD)

## Description

This invention relates to a method of producing keto acids.

Keto acids are useful intermediates for the production of fluoran compounds used as dyestuff in pressure- or heat-sensitive recording.

The keto acids have hitherto been produced by the reaction of an N,N-dialkyl-m-aminophenol with phthalic anhydride in a molar ratio of phthalic anhydride to N,N-dialkyl-m-aminophenol of 0.5-2 both dissolved in an inactive organic solvent such as toluene, xylene or tetrahydrofuran at a temperature of 80-150°C. However, the method provides a considerable amount of Rhodamines known as red dyes by the reaction of the resultant keto acids with the N,N-dialkyl-m-aminophenol, thereby to reduce the yield of the keto acids, as well as to make it difficult to obtain high purity keto acids.

To solve the above problem, there has been proposed a method in which an aqueous solution of an alkali metal hydroxide such as sodium hydroxide is added to the resultant reaction mixture, the reaction mixture is heated to decompose by-produced Rhodamines to alkali metal salts of the keto acid, the alkali metal salt of the keto acid is crystallized and then dissolved again in water, and then the salt is neutralized in water to recover the keto acid, as disclosed in Japanese Patent Application Laid-open No. 62-70350. However, the method needs many steps, and in addition, the method produces a large amount of neutralization waste water.

DE-A-8,5931 discloses in Example II the condensation of N,N-diethyl-m-aminophenol with phthalic anhydride in the presence of 4.54 parts by weight of toluene per part by weight of the N,N-diethyl-m-aminophenol. It is observed in the specification that the precipitation of crystals can be used as an indicator of completion of reaction. DE-A-8,5931 is referenced in GB-A-1,306,263 as describing the synthesis of a starting material for fluoran compound synthesis.

GB-A-1,182,743, which is also directed to fluoran compound synthesis, also discloses condensation of N,N-diethyl-m-aminophenol with phthalic anhydride in toluene to provide a starting material, the toluene being present in 3.93 parts by weight per part by weight of N,N-diethyl-m-aminophenol. The condensation product is disclosed as being gradually crystallised from solution.

The present invention seeks to provide a method of producing keto acids of high purity in high yield by the reaction of an N,N-dialkyl-m-aminophenol with phthalic anhydride while suppressing undesirable by-production of Rhodamines.

The present invention provides a method of producing a keto acid of the general formula (IA) 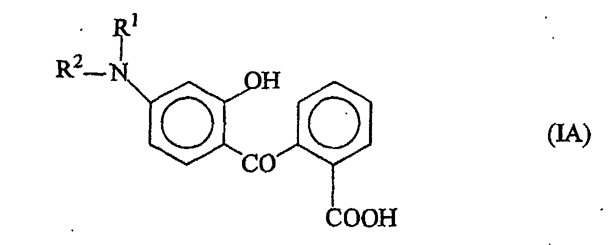 wherein R¹ and R² independently represent alkyl of 1-6 carbons or cycloalkyl of 4-8 carbons, except where R¹ and R² are both ethyl, which comprises reacting a m-aminophenol of the general formula 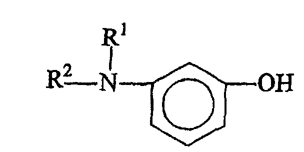 wherein R¹ and R² are as defined above in relation to formula (IA) with phthalic anhydride, at a temperature of from 60 to 120°C, in the presence of a controlled amount of an organic solvent to deposit the resultant keto acid in the solvent so that the reaction is effected in a slurry, provided that the amount of organic solvent is other than 0.5-3 parts by weight per one part by weight of the m-aminophenol, wherein said solvent is selected from aromatic hydrocarbons of 6 to 10 carbons, aliphatic hydrocarbons of 8 to 12 carbons, halogenated hydrocarbons of 2 to 8 carbons, tetrahydrofuran, dibutyl ether and diphenyl ether.

The present invention also provides as a method of producing a keto acid of the general formula (I) 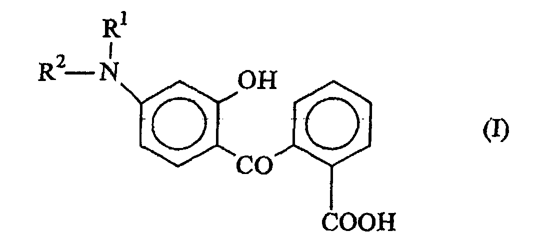 wherein R¹ and R² independently represent alkyl of 1-6 carbons or cycloalkyl of 4-8 carbons, which comprises reacting a m-aminophenol of the general formula 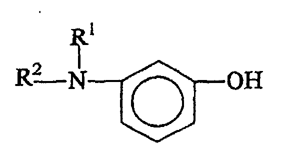 wherein R¹ and R² are as defined above in relation to formula (I) with phthalic anhydride, at a temperature of from 60 to 120°C, in the presence of a controlled amount of an organic solvent other than toluene to deposit the resultant keto acid in the solvent so that the reaction is effected in a slurry, provided that the amount of organic solvent is other than 0.5-3 parts by weight per one part by weight of the m-aminophenol, wherein said solvent is selected from aromatic hydrocarbons of 6 to 10 carbons, aliphatic hydrocarbons of 8 to 12 carbons, halogenated hydrocarbons of 2 to 8 carbons, tetrahydrofuran, dibutyl ether and diphenyl ether.

The m-aminophenol wherein both R¹ and R² are alkyls of 1-6 carbons used in the invention includes, for example, N,N-dimethyl-m-aminophenol, N,N-diethyl-m-aminophenol, N,N-di-n-propyl-m-aminophenol, N,N-di-isopropyl-m-aminophenol, N,N-di-n-butyl-m-aminophenol, N-methyl-N-ethyl-m-aminophenol. N-ethyl-N-isopropyl-m-aminophenol, N-ethyl-N-n-butyl-m-aminophenol and N-ethyl-N-isoamyl-m-aminophenol. The m-aminophenol wherein one of R¹ and R² is a cycloalkyl may be exemplified by N-ethyl-N-cyclohexyl-m-aminophenol.

For the reaction of the m-aminophenol derivative as above mentioned with phthalic anhydride, the latter is used usually in an amount of 0.7-2 moles per mole of the m-aminophenol derivative. The reaction is effected in a controlled amount of an organic solvent so that the resultant keto acid deposits in the solvent thereby to form a slurry, and the reaction is allowed to proceed in such a slurry. Strictly speaking, the amount of the solvent used is determined so that the resultant keto acid deposits in the solvent and thus the reaction proceeds in a slurry of the keto acid in the solvent. However, the amount of the solvent is other than 0.5-3 parts by weight in relation to one part by weight of the m-aminophenol derivative used.

The organic solvent used is selected from, an aromatic hydrocarbon of 6-10 carbons such as benzene, toluene or xylene, an aliphatic hydrocarbon of 8-12 carbons such as octane, isooctane or decane, a halogenated hydrocarbon of 2-8 carbons, aliphatic, cycloaliphatic or aromatic, such as perchlene or chlorobenzene and ethers such as tetrahydrofuran, dibutyl ether or diphenyl ether, among which are especially preferred aromatic hydrocarbons or ethers.

The reaction is effected, at a temperature in the range of 60-120°C for a period of 4-40 hours, although the reaction temperature and time are not critical. After the reaction, the reaction mixture is cooled usually to normal temperature, preferably to a temperarture of 0-35°C, most preferably to 10-30°C, depending upon the solvent used, or a bad solvent such as a saturated hydrocarbons is added to the reaction mixture, to effect primary crystallization of the resultant keto acid.

The resultant crude crystals are then dissolved under heating in an aliphatic alcohol of 1-4 carbons and then secondary crystallization is effected. The secondary crystallization may be effected at the same temperature range as in the primary crystailization. The secondary crystallization enables a high purity keto acid which contains substantially no Rhodamine impurities to be obtained.

There may be used as the alcohol for the secondary crystallization solvent, for example, methanol, ethanol, propanols such as isopropanol or butanols such as n-butanol. There may also be used a mixture of the alcohol with water, or a mixture of the alcohol with a hydrocarbon solvent, preferably an aromatic hydrocarbon of 6-10 carbons such as toluene or xylene, or an aliphatic hydrocarbon of 5-10 carbons such as pentane, hexane or heptane.

The crude crystals may be dissolved in the alcohol under an elevated pressure, usually under a pressure of several atmospheric pressures, and then the solution may be cooled to effect the secondary crystallization.

Further according to the invention, after the reaction, an aliphatic alcohol of 1-4 carbons may be added to the reaction mixture and then primary crystallization may be effected. The addition of the aliphatic alcohol to the reaction mixture enables selective dissolution of Rhodamines so that the slurry is kept in a good state from which the resultant crystals can be collected by filtration easily.

As above set forth, the reaction of the m-aminophenol derivative with phthalic anhydride is carried out in a controlled amount of an organic solvent to allow the resultant keto acid to deposit in the solvent and the reaction to proceed in a slurry according to the invention. Thus, the by-production of undesirable Rhodamines is effectively suppressed to improve the selectivity of the reaction to the keto acid. In addition, there is obtained a high purity keto acid which contains substantially no Rhodamine impurities by effecting secondary crystallization of the resultant keto acid out of the alcohol.

Further according to the invention, the alcohol may be recovered from the the secondary crystallization mother liquor, and if necessary, the alcohol is completely removed, to provide a residual solid which contains the keto acid. The solid is then dissolved in an inactive organic solvent which can be used as a reaction solvent, and the thus resultant solution may be added to the reaction mixture, and the mixture is then cooled to effect primary crystallization. This results in a remarkable improvement in yield of keto acid.

The amount of undesirable Rhodamines by-produced in the reaction is small according to the invention, and thus the ratio of the Rhodamines to the keto acid in the mother liquor after the secondary crystallization is very small. Therefore, according to the invention, the solvent in the mother liquor is exchanged with an organic solvent which can be used as a reaction solvent, and the resultant solution containing the keto acid can be advantageously used for primary crystallization together with the reaction mixture, thereby to increase the yield of the keto acid.

The invention will now be described in more detail with reference to examples, however, the invention is not limited to the examples.

### Reference Example 1

An amount of 165 g (1.0 mole) of N,N-diethyl-m-amino-phenol, 170.3 g (1.15 mole) of phthalic anhydride and 206 g of xylene were placed in a reactor, and stirred for 7 hours at 115°C while allowing the resultant keto acid (4-N,N-diethylamino-2-hydroxy-2'-carboxybenzophenone) to deposit in the reaction mixture and the reaction to proceed in a slurry.

After the reaction, 247 g of xylene were added to the reaction mixture, and the mixture was cooled gradually to 20°C so that the keto acid crystalized out. The crystals were collected by filtration and washed with 577 g of n-butanol to provide 303.9 g of crude crystals.

An amount of 1486 g of n-butanol was added to the crystals and heated to dissolve the crystals therein, and then the mixture was cooled to 20°C gradually. The resultant crystals were collected by filtration and dried to provide 291.9 g of high purity keto acid (4-N,N-diethyl-amino-2-hydroxy-2'-carboxybenzophenone). The amount of Rhodamines in the keto acid was found to be not more than 0.1% by liquid chromatographic analysis. The yield was 93.1 mol %.

### Reference Example 2

After the reaction, n-butanol was added to the reaction mixture in place of xylene, and otherwise in the same manner as in Example 1, crude crystals were obtained.

An amount of 1486 g of n-butanol was added to 303.6 g of the crystals and heated to dissolve the crystals therein, and then the mixture was cooled to 20°C gradually. The resultant pale yellow crystals were collected by filtration and dried to provide 291.5 g of high purity keto acid (4-N,N-diethylamino-2-hydroxy-2'-carboxybenzophenone).

No Rhodamines were detected in the keto acid by liquid chromatographic analysis. The yield was 93.5 mol %.

### Reference Example 3

The reaction was effected in toluene in place of xylene, and after the reaction methanol was added in place of xylene to the reaction mixture, and the mixture was cooled gradually to 20°C so that the keto acid crystalized out and the crystals were washed with methanol, and otherwise in the same manner as in Example 1.

An amount of 913 g of methanol was added to 304.5 g of the crude crystals and heated to 113°C under a pressure of 3 Kg/cm² to completely dissolve the crystals therein, and then the mixture was cooled to 20°C gradually. The resultant pale yellow crystals were collected by filtration and dried to provide 295.3 g of high purity keto acid (4-N,N-diethylamino-2-hydroxy-2'-carboxybenzophenone).

No Rhodamines were detected in the keto acid by liquid chromatographic analysis. The yield was 94.3 mol %.

### Reference Example 4

An amount of 221 g (1.0 mole) of N,N-di-n-butyl-m-aminophenol, 177.7 g (1.2 mole) of phthalic anhydride and 220 ml of xylene were placed in a reactor, and stirred for 7 hours at 100°C while allowing the resultant keto acid (4-N,N-di-n-butylamino-2-hydroxy-2'-carboxybenzophenone) to deposit in the reaction mixture and the reaction to proceed in a slurry.

After the reaction, 220 ml of xylene were added to the reaction mixture, and the mixture was cooled gradually to 20°C so that the keto acid crystalized out. The crystals were collected by filtration and washed with 80 ml of xylene twice.

An amount of 320 g of the resultant crude crystals was added to 1250 g of methanol. The mixture was heated to dissolve the crystals in the methanol, and then the mixture was cooled to 20°C gradually to effect recrystallization of the keto acid. The resultant crystals were collected by filtration and washed with 100 g of cold methanol twice followed by drying the crystals to provide 255 g of high purity keto acid (4-N,N-di-n-butylamino-2-hydroxy-2'-carboxybenzophenone).

No Rhodamines were detected in the keto acid by liquid chromatographic analysis. The yield was 69.0 mol %.

### Reference Example 5

The reaction was effected in toluene in place of xylene, and otherwise in the same manner as in Example 4 to provide 258 g of high purity keto acid (4-N,N-di-n-butylamino-2-hydroxy-2'-carboxybenzophenone).

No Rhodamines were detected in the keto acid by liquid chromatographic analysis. The yield was 69.8 mol %.

### Reference Example 6

The crystals of keto acid (4-N,N-di-n-butylamino)-2-hydroxy-2'-carboxybenzophenone) obtained in Example 4 were recrystallized, collected by filtration, and washed with cold methanol. The mother liquor was distilled to recover methanol,

An amount of 220 ml of xylene was added to the distillation bottom containing the keto acid to dissolve the keto acid therein.

The resultant solution was added to the same reaction mixture as obtained in Example 1 and then primary crystallization was effected, followed by working in the same manner as in Example 1, there were obtained 299 g of pale yellow crystals of high purity keto acid (4-N,N-di-n-butylamino-2-hydroxy-2'-carboxybenzophenone).

No Rhodamines were detected in the keto acid by liquid chromatographic analysis. The yield was 81 mol % based on the N,N-di-n-butyl-m-aminophenol used.

### Reference Example 7

An amount of 207 g (1.0 mole) of N-ethyl-N-isoamyl-m-aminophenol, 177.6 g (1.2 mole) of phthalic anhydride and 300 g of diphenyl ether were placed in a reactor, and stirred for 35 hours at 60°C while allowing the resultant keto acid (4-N-ethyl-N-isoamylamino-2-hydroxy-2'-carboxybenzophenone) to deposit in the reaction mixture and the reaction to proceed in a slurry.

The conversion rate of N-ethyl-N-isoamyl-m-aminophenol was 66%; the yield of keto acid (4-N-ethyl-N-isoamyiamino-2-hydroxy-2'-carboxybenzophenone) was 65%; and the yield of Rhodamines was 1%.

After the reaction, the reaction mixture was cooled to 30°C so that the keto acid crystalized out, and the crystals were collected by filtration. An amount of 1700 ml of a mixture of methanol/water (75/25 in volume ratio) was added to 230 g of the crystals and heated to dissolve the crystals therein. The mixture was then cooled gradually to 20°C to effect recrystallization and the resultant crystals were collected by filtration.

The amount of Rhodamines in the keto acid was found to be not more than 0.1% by liquid chromatographic analysis.

## Claims

1. A method of producing a keto acid of the general formula (IA) 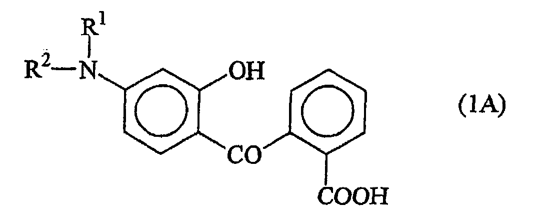 wherein R¹ and R² independently represent alkyl of 1-6 carbons or cycloalkyl of 4-8 carbons, except where R¹ and R² are both ethyl, which comprises reacting a m-aminophenol of the general formula 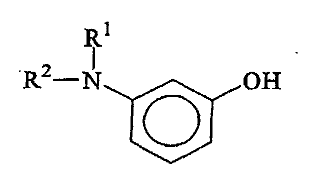 wherein R¹ end R² are as defined above in relation to formula (IA) with phthalic anhydride, at a temperature of from 60 to 120°C, in the presence of a controlled amount of an organic solvent to deposit the resultant keto acid in the solvent so that the reaction is effected in a slurry, provided that the amount of organic solvent is other than 0.5-3 parts by weight per one part by weight of the m-aminophenol, wherein said solvent is selected from aromatic hydrocarbons of 6 to 10 carbons, aliphatic hydrocarbons of 8 to 12 carbons, halogenated hydrocarbons of 2 to 8 carbons, tetrahydrofuran, dibutyl ether and diphenyl ether.

2. A method of producing a keto acid of the general formula (I) 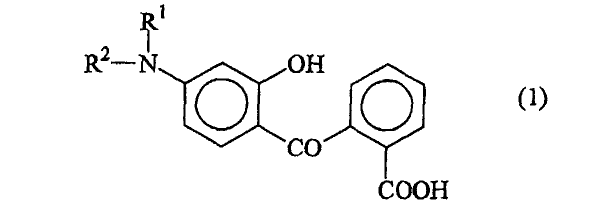 wherein R¹ and R² independently represent alkyl of 1-6 carbons or cycloalkyl of 4-8 carbons, which comprises reacting a m-aminophenol of the general formula 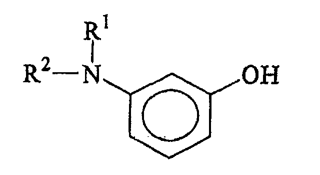 wherein R¹ and R² are as defined above in relation to formula (I) with phthalic anhydride, at a temperature of from 60 to 120°C, in the presence of a controlled amount of an organic solvent other than toluene to deposit the resultant keto acid in the solvent so that the reaction is effected in a slurry, provided that the amount of organic solvent is other than 0.5-3 parts by weight per one part by weight of the m-aminophenol, wherein said solvent is selected from aromatic hydrocarbons of 6 to 10 carbons, aliphatic hydrocarbons of 8 to 12 carbons, halogenated hydrocarbons of 2 to 8 carbons, tetrahydrofuran, dibutyl ether and diphenyl ether.

3. A method according to claim 1, wherein the organic solvent is benzene, toluene, xylene or diphenyl ether.

4. A method according to claim 2, wherein the organic solvent is benzene, xylene or diphenyl ether.

5. A method according to any one of claims 1 to 4, which additionally comprises the steps of
(a) cooling the reaction mixture to effect primary crystallization to provide crude crystals of the keto acid;
(b) dissolving the crude crystals in an aliphatic alcohol of 1-4 carbons, or a mixture of the alcohol with water, or a mixture of the alcohol with a hydrocarbon solvent;
(c) effecting secondary crystallization from said alcohol or mixture;
(d) recovering said alcohol or mixture from the resulting crystallization mother liquor; and
(e) adding the recovered keto acid from the mother liquor to the reaction mixture for use in the primary crystallization.

6. A method according to claim 5, wherein in step (a) an aliphatic alcohol of 1-4 carbons is added to the reaction mixture, and then the primary crystallization is effected.

7. A method according to claim 5 or 6, wherein the aliphatic alcohol used in step (a) and/or (b) is methanol or butanol.

8. A method according to any one of claims 5 to 7, wherein the hydrocarbon solvent is an aromatic hydrocarbon of 6-10 carbons or an aliphatic hydrocarbon of 5-10 carbons.

9. A method according to claim 8, wherein the hydrocarbon solvent is toluene, xylene or hexane.

10. A method according to any one of claims 5 to 9, wherein in step (b) the crude crystals are dissolved in said alcohol or mixture under an elevated pressure.

## Patentansprüche

1. Verfahren zur Herstellung einer Ketosäure der allgemeinen Formel (IA) 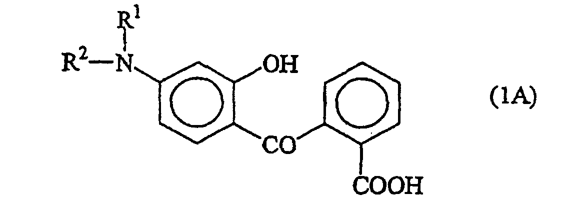 in der R¹ und R² unabhängig Alkyl mit 1 bis 6 Kohlenstoffatomen oder Cycloalkyl mit 4 bis 8 Kohlenstoffatomen bedeuten, außer wenn R¹ und R² beide Ethyl sind, umfassend das Umsetzen eines m-Aminophenols der allgemeinen Formel 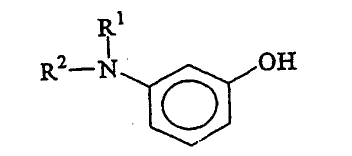 in der R¹ und R² wie oben im Zusammenhang mit der Formel (IA) definiert sind, mit Phathalsäure-anhydrid bei einer Temperatur von 60 bis 120°C, in Gegenwart einer kontrollierten Menge eines organischen Lösungsmittels, um die entstandene Ketosäure in dem Lösungsmittel auszufällen, so daß die Reaktion in Aufschlämmung durchgeführt wird, mit der Maßgabe, daß die Menge an organischem Lösungsmittel nicht 0,5 bis 3 Gew.-Teile pro 1 Gew.-Teil des m-Aminophenols beträgt, wobei das Lösungsmittel ausgewählt ist aus aromatischen Kohlenwasserstoffen mit 6 bis 10 Kohlenstoffatomen, aliphatischen Kohlenwasserstoffen mit 8 bis 12 Kohlenstoffatomen, halogenierten Kohlenwasserstoffen mit 2 bis 8 Kohlenstoffatomen, Tetrahydrofuran, Dibutylether und Diphenylether.

2. Verfahren zur Herstellung einer Ketosäure der allgemeinen Formel (I) 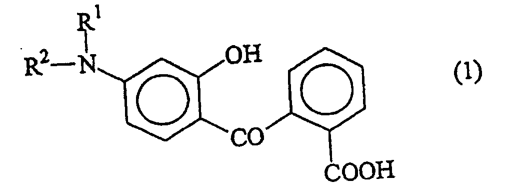 in der R¹ und R² unabhängig Alkyl mit 1 bis 6 Kohlenstoffatomen oder Cycloalkyl mit 4 bis 8 Kohlenstoffatomen bedeuten, umfassend das Umsetzen eines m-Aminophenols der allgemeinen Formel 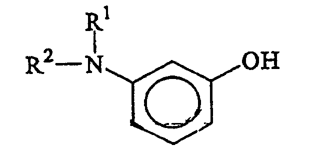 in der R¹ und R² wie oben im Zusammenhang mit der Formel (I) definiert sind, mit Phathalsäure-anhydrid bei einer Temperatur von 60 bis 120°C, in Gegenwart einer kontrollierten Menge eines organischen Lösungsmittels, außer Toluol, um die entstandene Ketosäure in dem Lösungsmittel auszufällen, so daß die Reaktion in Aufschlämmung durchgeführt wird, mit der Maßgabe, daß die Menge an organischem Lösungsmittel nicht 0,5 bis 3 Gew.-Teile pro 1 Gew.-Teil des m-Aminophenols beträgt, wobei das Lösungsmittel ausgewählt ist aus aromatischen Kohlenwasserstoffen mit 6 bis 10 Kohlenstoffatomen, aliphatischen Kohlenwasserstoffen mit 8 bis 12 Kohlenstoffatomen, halogenierten Kohlenwasserstoffen mit 2 bis 8 Kohlenstoffatomen, Tetrahydrofuran, Dibutylether und Diphenylether.

3. Verfahren nach Anspruch 1, wobei das organische Lösungsmittel Benzol, Toluol, Xylol oder Diphenylether ist.

4. Verfahren nach Anspruch 2, wobei das organische Lösungsmittel Benzol, Xylol oder Diphenylether ist.

5. Verfahren nach einem der Ansprüche 1 bis 4, das zusätzlich die folgenden Stufen umfaßt:
a) Kühlen des Reaktionsgemisches, um eine erste Kristallisation zur Bildung von rohen Kristallen der Ketosäure hervorzurufen;
b) Lösen der rohen Kristalle in einem aliphatischen Alkohol mit 1 bis 4 Kohlenstoffatomen oder einem Gemisch des Alkohols mit Wasser oder einem Gemisch des Alkohols mit einem Kohlenwasserstoff-Lösungsmittel;
c) Bewirken einer zweiten Kristallisation aus dem Alkohol oder dem Gemisch;
d) Gewinnen des Alkohols oder des Gemisches aus der erhaltenen Kristallisations-Mutterlauge und
e) Zugeben der aus der Mutterlauge gewonnenen Ketosäure zu dem Reaktionsgemisch zur Verwendung bei der ersten Kristallisation.

6. Verfahren nach Anspruch 5, wobei in Stufe a) ein aliphatischer Alkohol mit 1 bis 4 Kohlenstoffatomen zu dem Reaktionsgemisch zugesetzt und dann die erste Kristallisation durchgeführt wird.

7. Verfahren nach Anspruch 5 oder 6 wobei der in Stufe a) und/oder b) verwendete Alkohol Methanol oder Butanol ist.

8. Verfahren nach einem der Ansprüche 5 bis 7, wobei das Kohlenwasserstoff-Lösungsmittel ein aromatischer Kohlenwasserstoff mit 6 bis 10 Kohlenstoffatomen oder ein aliphatischer Kohlenwasserstoff mit 5 bis 10 Kohlenstoffatomen ist.

9. Verfahren nach Anspruch 8, wobei das Kohlenwasserstoff-Lösungsmittel Toluol, Xylol oder Hexan ist.

10. Verfahren nach einem der Ansprüche 5 bs 9, wobei in Stufe b) die rohen Kristalle in dem Alkohol oder dem Gemisch unter erhöhtem Druck gelöst werden.

## Revendications

1. Procédé de production d'un acide cétonique de formule générale (IA) : 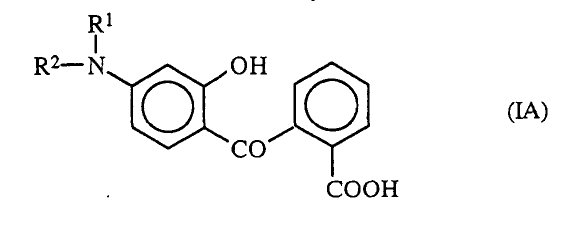 dans laquelle R¹ et R² représentent chacun indépendamment un groupe alkyle ayant 1 à 6 atomes de carbone ou un groupe cycloalkyle ayant 4 à 8 atomes de carbone, R¹ et R² n'étant pas tous les deux un groupe éthyle, qui comprend la réaction d'un m-aminophénol de formule générale 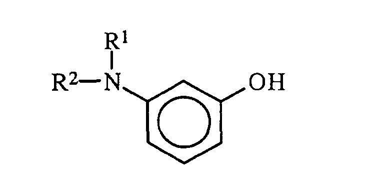 dans laquelle R¹ et R² sont tels que définis précédemment pour la formule (IA), avec de l'anhydride phtalique, à une température de 60 à 120 °C, en présence d'une quantité réglée d'un solvant organique pour faire précipiter l'acide cétonique résultant dans le solvant, de sorte que la réaction est effectuée dans une suspension, étant entendu que la quantité de solvant organique est autre que 0,5 à 3 parties en poids pour une partie en poids de m-aminophénol, ledit solvant étant choisi parmi les hydrocarbures aromatiques ayant 6 à 10 atomes de carbone, les hydrocarbures aliphatiques ayant 8 à 12 atomes de carbone, les hydrocarbures halogénés ayant 2 à 8 atomes de carbone, le tétrahydrofurane, l'oxyde de dibutyle et l'oxyde de diphényle.

2. Procédé de production d'un acide cétonique de formule générale (I) : 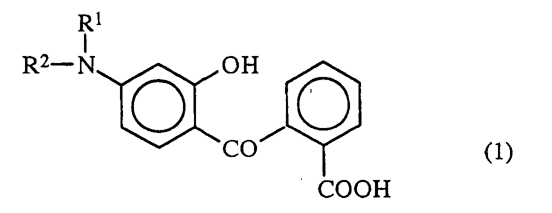 dans laquelle R¹ et R² représentent chacun indépendamment un groupe alkyle ayant 1 à 6 atomes de carbone ou un groupe cycloalkyle ayant 4 à 8 atomes de carbone, qui comprend la réaction d'un m-aminophénol de formule générale 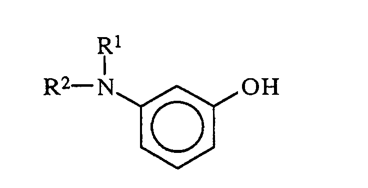 dans laquelle R¹ et R² sont tels que définis précédemment pour la formule (I) avec de l'anhydride phtalique, à une température de 60 à 120 °C, en présence d'une quantité réglée d'un solvant organique autre que le toluène, pour faire précipiter l'acide cétonique résultant dans le solvant de sorte que la réaction est effectuée dans une suspension, étant entendu que la quantité de solvant organique est autre que 0,5 à 3 parties en poids pour une partie en poids de m-aminophénol, ledit solvant étant choisi parmi les hydrocarbures aromatiques ayant 6 à 10 atomes de carbone, les hydrocarbures aliphatiques ayant 8 à 12 atomes de carbone, les hydrocarbures halogénés ayant 2 à 8 atomes de carbone, le tétrahydrofurane, l'oxyde de dibutyle et l'oxyde de diphényle.

3. Procédé selon la revendication 1, dans lequel le solvant organique est le benzène, le toluène, le xylène ou l'oxyde de diphényle.

4. Procédé selon la revendication 2, dans lequel le solvant organique est le benzène, le xylène ou l'oxyde de diphényle.

5. Procédé selon l'une quelconque des revendications 1 à 4, qui comprend en outre les étapes consistant à :
(a) refroidir le mélange réactionnel pour effectuer une cristallisation primaire pour obtenir des cristaux bruts de l'acide cétonique,
(b) dissoudre les cristaux bruts dans un alcool aliphatique ayant 1 à 4 atomes de carbone ou dans un mélange d'un tel alcool avec de l'eau ou dans un mélange d'un tel alcool avec un solvant hydrocarboné,
(c) effectuer une cristallisation secondaire à partir dudit alcool ou dudit mélange,
(d) récupérer ledit alcool ou ledit mélange à partir de la liqueur mère de cristallisation résultante, et
(e) ajouter l'acide cétonique récupéré à partir de la liqueur mère au mélange réactionnel destiné à être utilisé dans la cristallisation primaire.

6. Procédé selon la revendication 5, dans lequel, dans l'étape (a), on ajoute au mélange réactionnel un alcool aliphatique ayant 1 à 4 atomes de carbone, et on effectue ensuite la cristallisation primaire.

7. Procédé selon la revendication 5 ou 6, dans lequel l'alcool aliphatique utilisé dans l'étape (a) et/ou l'étape (b) est le méthanol ou le butanol.

8. Procédé selon l'une quelconque des revendications 5 à 7, dans lequel le solvant hydrocarboné est un hydrocarbure aromatique ayant 6 à 10 atomes de carbone ou un hydrocarbure aliphatique ayant 5 à 10 atomes de carbone.

9. Procédé selon la revendication 8, dans lequel le solvant hydrocarboné est le toluène, le xylène ou l'hexane.

10. Procédé selon l'une quelconque des revendications 5 à 9, dans lequel, dans l'étape (b), les cristaux bruts sont dissous dans ledit alcool ou ledit mélange sous une pression élevée.
